# EUROPÄISCHE PATENTSCHRIFT

(11) **EP 0 614 890 B1**
(45) Veröffentlichungstag und Bekanntmachung des Hinweises auf die Patenterteilung: **03.09.1997**
(21) Anmeldenummer: 94103636.0
(22) Anmeldetag: 09.03.1994
(51) Int. Cl.: C07D 233/68

(54) **Verfahren zur Herstellung von gegebenfalls 2-substituierten 5-Chlorimidazolen**
Process for preparation of optionally 2-substituted 5-chloroimidazoles
Procédé pour la préparation de 5-chloroimidazoles facultativement substitués sur la position 2

(30) Priorität: 12.03.1993 CH 748/93
(43) Veröffentlichungstag der Anmeldung: 14.09.1994
(73) Patentinhaber: Lonza AG, 4002 Basel (CH)
(72) Erfinder: Griffiths, Gareth, Dr., Visp (Kanton Wallis) (CH); Imwinkelried, René, Dr., Brig-Glis (Kanton Wallis) (CH); Gosteli, Jacques, Dr., Basel (CH)
(74) Vertreter: Weinhold, Peter, Dr.

(56) Entgegenhaltungen:
- EP-A- 0 028 834
- EP-A- 0 365 030
- EP-A- 0 429 257
- EP-A- 0 479 479
- EP-A- 0 505 098
- DE-A- 2 804 435
- DE-A- 3 145 927
- DE-A- 3 330 192
- US-A- 3 409 606
- JOURNAL OF HETEROCYCLIC CHEMISTRY, Bd.27, Nr.3, 1990 Seiten 711 - 716 T. WATANABE ET AL.
- JOURNAL OF HETEROCYCLIC CHEMISTRY, Bd.4, 1967 Seiten 399 - 402 A.W. LUTZ ET AL.
- JOURNAL OF HETEROCYCLIC CHEMISTRY, Bd.4, 1967 Seiten 451 - 452 J.L. IMBACH ET AL.
- JOURNAL OF THE CHEMICAL SOCIETY, PERKIN TRANSACTIONS I, Nr.7, 1980 Seiten 2310 - 2315 T. BROWN ET AL.
- HETEROCYCLES, Bd.35, Nr.1, 1993 Seiten 121 - 124 D.H. BOSCHELLI ET AL.
- PATENT ABSTRACTS OF JAPAN vol. 12, no. 117 (C-487) 13. April 1988 & JP-A-62 238 383 (CHIYODA KAGAKU KENKYUSHO K.K.) 19. Oktober 1987
- BULLETIN DE LA SOCIETE CHIMIQUE DE FRANCE, Nr.3, 1971 Seiten 1040 - 1051 R. JACQUIER ET AL.
- SYNTH.COMMUN, 1992, 22 (20), Seiten 2971-7

## Beschreibung

Die Erfindung betrifft ein Verfahren zur Herstellung von gegebenenfalls 2-substituierten 5-Chlorimidazolen der allgemeinen Formel 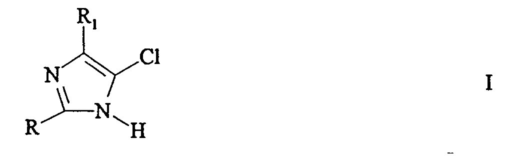 worin R Wasserstoff, eine geradkettige oder verzweigte C₁-C₆-Alkylgruppe, eine geradkettige oder verzweigte C₂-C₆-Alkenylgruppe, eine Cyclopropyl-, Cyclobutyl-, Cyclopentyl- oder Cyclohexylgruppe, oder eine gegebenenfalls mit Halogenatomen, Alkyl-, Nitro- und/oder Aminogruppen substituierte Benzylgruppe oder Phenylgruppe bedeutet und R₁ Wasserstoff, eine geradkettige oder verzweigte C₁-C₆-Alkylgruppe, eine Cyclopropyl-, Cyclobutyl-, Cyclopentyl- oder Cyclohexylgruppe, oder eine gegebenenfalls mit Halogenatomen, Alkyl-, Nitro- und/oder Aminogruppen substituierte Benzylgruppe oder Phenylgruppe bedeutet.

Verbindungen der Formel I, in denen R₁ Wasserstoff ist, können beispielsweise zu gegebenenfalls 2-substituierten 5-Chlorimidazol-4-carbaldehyden der allgemeinen Formel 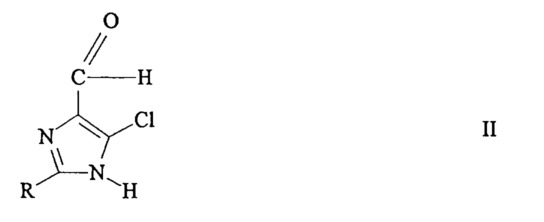 umgesetzt werden, worin R die oben genannte Bedeutung hat. Die gegebenenfalls 2-substituierten 5-Chlorimidazole der allgemeinen Formel I und die gegebenenfalls 2-substituierten 5-Chlorimidazol-4-carbaldehyde der allgemeinen Formel II sind wichtige Ausgangsprodukte zur Herstellung von blutdrucksenkenden Pharmazeutika (US-A-4 355 040) oder von herbizid-wirksamen Verbindungen (DE-A-28 04 435).

Watanabe, T., et al. (J. Heterocycl. Chem., 1990, 27, 711-716) beschreiben die Herstellung von substituierten Chlorimidazolen ausgehend von 2,5-Dichlorpyrazinen.

Die DE-A-34 09 606 offenbart ein Herstellungsverfahren für Chlorimidazole, bei dem man Imidazole mit einem Alkalihypochlorit in basischem Medium umsetzt. Ein analoges Verfahren wird auch von A.W. Lutz und S. DeLorenzo (J. Heterocycl. Chem., 1967, 4, 399-402) beschrieben.

Imbach, J.L., et al. (J. Heterocycl. Chem., 1967, 4, 451-452) beschreiben ein Verfahren zur Herstellung von Chlorimidazolen durch direkte Chlorierung von Imidazol mit N-Chlorsuccinimid. Eine Chlorierung von Imidazol mit N-Chlorsuccinimid wird auch von D.H. Boschelli und D.T. Connor (Heterocycles, 1993, 35, 121-124) beschrieben.

Die EP-A-0 505 098 und die EP-A-0 479 479 beschreiben substituierte Chlorimidazole als Ausgangsprodukte für die Herstellung von Angiotensin II-Antagonisten. In der EP-A-0 429 257 werden halogensubstituierte Imidazole als Ausgangsprodukte für die Herstellung von Indolderivaten beschrieben, die beispielsweise zur Behandlung und Prophylaxe von Bluthochdruck eingesetzt werden können. Keine der genannten Druckschriften offenbart das erfindungsgemäße Verfahren zur Herstellung von gegebenenfalls 2-substituierten 5-Chlorimidazolen.

Die EP-A-0 365 030 betrifft Imidazole, die an 2-Position mit Carboxylgruppen oder deren Derivaten substituiert sind. Die DE-A-31 45 927 offenbart 4-Methyl-5-Chlorimidazole. Die Verfahren, die zur Herstellung der genannten Verbindungen vorgeschlagen werden, unterscheiden sich jedoch deutlich von dem Verfahren, wie es erfindungsgemäß zur Herstellung von gegebenenfalls 2-substituierten 5-Chlorimidazolen zur Anwendung kommt.

Brown, T., et al. (J. Chem. Soc., Perkin Trans. I, 1980, (7), 2310-15) beschreiben die Herstellung von 5-Chlorimidazol-4-ethylcarboxylat durch Diazotierung von 5-Aminoimidazol-4-ethylcarboxylat in Gegenwart von Kupferchlorid.

Die DE-A-33 30 192 beschreibt 1-N-Carbamoyl-5-Chlorimidazole, die als Nitrifikationshemmer geeignet sind und durch Umsetzung von 5-Chlorimidazolen mit Säurechloriden oder Isocyanaten hergestellt werden. Die Herstellung der 5-Chlorimidazole wird nicht beschrieben.

Die JP-A-62 238383 [wie zitiert in Pat. Abs. Japan, 1988, 12, No. 117 (C-487)] beschreibt Chlorimidazole, die als Rostschutzmittel verwendet werden. Es findet sich kein Hinweis auf das erfindungsgemäße Verfahren.

Zur Herstellung der gegebenenfalls 2-substituierten 5-Chlorimidazol-4-carbaldehyde sind ebenfalls mehrere Wege bekannt.

So beschreibt die US-A-4 355 040 ein Verfahren, nach welchem 2-Amino-3,3-dichloracrylnitril mit einem Aldehyd zum entsprechenden Azomethinzwischenprodukt und weiter mit einem Halogenwasserstoff und Wasser zum 2-substituierten 5-Halogenimidazol-4-carbaldehyd umgesetzt wird. Experimentelle Angaben fehlen in der genannten Patentschrift. Ein großer Nachteil der Synthese besteht aber darin, daß das eingesetzte 2-Amino-3,3-dichloracrylnitril zunächst ausgehend von Dichloracetonitril durch dessen Umsetzung mit Blausäure/Natriumcyanid hergestellt werden muß. Die äußerst toxischen Reaktionsteilnehmer und die damit verbundenen sicherheitstechnischen Maßnahmen, die bereits für die Bereitstellung des Ausgangsproduktes notwendig sind, machen das Gesamtverfahren industriell untauglich.

Die US-A-4 355 040 offenbart in einer weiteren Variante ein 3-Stufen Verfahren, worin in einer ersten Stufe ein Amidinhydrochlorid mit Dihydroxyaceton mit hohem NH₃-Druck ringgeschlossen wird, der Imidazolalkohol halogeniert wird und schließlich zum Aldehyd oxidiert wird. Es hat sich gezeigt, daß für die Ringschlußreaktion Drucke von über 20 bar notwendig sind. Die Oxidation des Alkohols funktioniert gemäß US-A-4 355 040 in Gegenwart von Chromoxid.

Es ist offensichtlich, daß eine Oxidation mit Schwermetalloxiden, die in der Regel nicht rezirkulierbar sind, nach heutigen ökologischen Gesichtspunkten nicht mehr verantwortbar ist.

Aufgabe der vorliegenden Erfindung war es, ein neues Verfahren zur Herstellung von gegebenenfalls 2-substituierten 5-Chlorimidazolen der allgemeinen Formel I bereitzustellen, die als Ausgangsprodukte zur Herstellung von Pharmazeutika oder herbizidwirksamen Verbindungen verwendet werden können.

Diese Aufgabe wurde durch ein Verfahren gemäß Anspruch 1 gelöst.

Mit Hilfe des erfindungsgemäßen Verfahrens können gleichzeitig neue Verbindungen der Formel I hergestellt werden. Diese Verbindungen sind ebenfalls Gegenstand der vorliegenden Erfindung.

Zur Herstellung der gegebenenfalls 2-substituierten 5-Chlorimidazole der allgemeinen Formel I wird in einer ersten Stufe ein Glycinesterhydrohalogenid der allgemeinen Formel 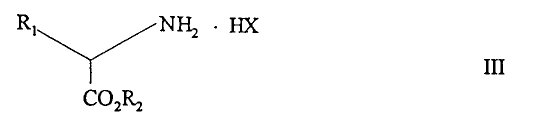 worin R₁ die genannte Bedeutung hat, R₂ für eine Alkylgruppe steht und X für ein Halogenatom steht mit einem Imidsäureester der allgemeinen Formel 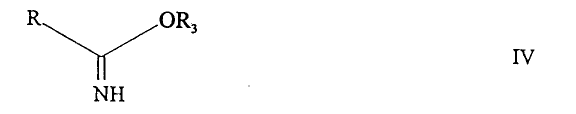 worin R die genannte Bedeutung hat und R₃ eine Alkylgruppe bedeutet in Gegenwart einer Base zum entsprechenden gegebenenfalls 2-substituierten 3,5-Dihydroimidazol-4-on der allgemeinen Formel 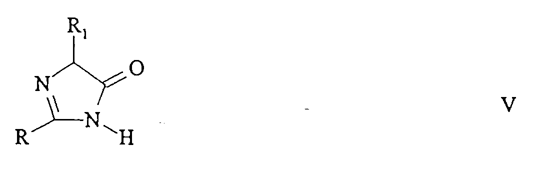 worin R und R₁ die genannte Bedeutung haben, umgesetzt.

In den allgemeinen Substituenten R, R₁, R₂ und R₃ haben die angegebenen Gruppen folgende Bedeutung:

Unter einer Alkylgruppe werden geradkettige oder verzweigte C₁-C₆-Alkylgruppen, wie z.B. Methyl, Ethyl-, n-Propyl, Isopropyl-, n-Butyl, sec. Butyl-, tert. Butyl-, Pentyl- oder Hexylgruppen verstanden. Bevorzugte Alkylgruppe ist eine der genannten C₁-C₄-Alkylgruppen. Bevorzugt für Substituent R ist die n-Butylgruppe.

Unter einer Alkenylgruppe wird eine geradkettige oder verzweigte C₂-C₆-Alkenylgruppe, wie z.B. 1-Propenyl, 2-Propenyl, 1-Butenyl, 2-Butenyl, 3-Butenyl, Pentenyl und seine Isomeren oder Hexenyl und seine Isomeren, verstanden. Für R ist die 2- oder 3-Butenyl-Gruppe bevorzugt.

Als Vertreter von Cycloalkylgruppen seien die Cyclopropyl-, die Cyclobutyl, die Cyclopentyl- oder die Cyclohexylgruppe genannt.

Sowohl die Benzyl- als auch die Phenylgruppe kann Substituenten, wie die genannten Alkylgruppen, Halogenatome, Nitrogruppen oder Aminogruppen, enthalten.

Unter der Bezeichnung Halogen wird zweckmässig Chlor, Brom oder Jod, vorzugsweise Chlor, verstanden.

Zweckmässig wird so vorgegangen, dass das Glycinesterhydrohalogenid der allgemeinen Formel III in Gegenwart einer Base zweckmässig bei einem pH-Wert von 7 bis 12, vorzugsweise von 9 bis 11, mit dem Imidsäureester der allgemeinen Formel IV reagiert wird.

Die Glycinesterhydrohalogenide der allgemeinen Formel III sind im Handel erhältliche stabile Verbindungen.

Geeignete Basen sind die Alkalihydroxide, wie z.B. Natriumhydroxid oder Kaliumhydroxid oder Alkalialkoholate, wie z.B. Natrium- oder Kaliummethylat, -ethylat oder -tert. butylat.

Vorteilhaft liegt die Base in einem geeigneten Lösungsmittel gelöst vor. Besonders geeignet sind aliphatische Alkohole wie Methanol oder Ethanol. Der Imidsäureester wird zweckmässig ebenfalls in Form einer Lösung in einem inerten Lösungsmittel zugegeben. In der Regel eignen sich hierzu aromatische Lösungsmittel, wie z.B. Toluol oder Chlorbenzol oder die genannten aliphatischen Alkohole besonders gut.
Von Vorteil erfolgt die Umsetzung der Reaktionsteilnehmer Glycinesterhydrohalogenid, Imidsäureester und Base im stöchiometrischen Verhältnis 1:1:1.

Die Umsetzungstemperatur rangiert zweckmässig im Bereich von -20°C bis 50°C, vorzugsweise bei 0° bis 25°C.

Nach einer Umsetzungszeit von wenigen Stunden kann das entsprechende 3,5-Dihydroimidazolin-4-on der allgemeinen Formel V auf fachmännische Weise, in der Regel durch einfache Filtration, in Ausbeuten von grösser als 95%, isoliert werden.

Von Vorteil wird das resultierende Reaktionsgemisch ohne Isolierung des gegebenenfalls 2-substituierten 3,5-Dihydroimidazolin-4-ons für die Weiterumsetzung zum entsprechenden 5-Chlorimidazol bereitgestellt (Eintopfverfahren).

Diese erste Stufe des erfindungsgemässen Verfahrens beinhaltet eine sprunghafte Verbesserung des bekannten Verfahrens nach R. Jacquier et al. (Bull. Soc. Chim. France, 1971, 1040), welches die Umsetzung des freien Glycinesters mit einem Imidsäureethylester in Abwesenheit eines Lösungsmittels zum entsprechenden 3,5-Dihydroimidazol-4-on umfasst. Nachteilig bei diesem bekannten Verfahren ist, dass der freie Glycinester sehr instabil ist und daher für jede Umsetzung jeweils neu synthetisiert und isoliert werden muss. Gemäss dem bekannten Verfahren konnten nach einer Umsetzungszeit von 24 Std. und länger Ausbeuten von lediglich 30% bis 48% erhalten werden.

In der zweiten Stufe wird das 3,5-Dihydroimidazol-4-on der allgemeinen Formel V zum entsprechenden 5-Chlorimidazol der allgemeinen Formel I chloriert.

Zweckmässig erfolgt die Chlorierung mit Thionylchlorid oder Phosphoroxychlorid, vorteilhaft in einem Überschuss an Chlorierungsmittel von 10 bis 300% bei einer Reaktionstemperatur im Bereich zwischen 20°C und 110°C. Das Chlorierungsmittel kann dabei zugleich als Lösungsmittel dienen, so dass ein zusätzliches Lösungsmittel in der Regel nicht notwendig ist. Bevorzugt wird Phosphoroxychlorid als Chlorierungsmittel angewendet. Das resultierende gegebenenfalls 2-substituierte 5-Chlorimidazol der allgemeinen Formel I kann auf fachmännische Weise, vorteilhaft durch Extraktion, in hoher Reinheit aus dem Reaktionsgemisch isoliert werden.

Bevorzugte 2-substituierte 5-Chlorimidazole der allgemeinen Formel I sind solche, worin R die Bedeutung von n-Butyl, 2-Butenyl oder 3-Butenyl hat.

Verbindungen der allgemeinen Formel I, worin R₁ Wasserstoff bedeutet, können wie erwähnt weiter zu den gegebenenfalls 2-substituierten 5-Chlorimidazol-4-carbaldehyden der Formel II umgesetzt werden. Eine solche Umsetzung kann zweckmäßig nach dem in der EP-A-0 614 891 beschriebenen Verfahren durchgeführt werden. Dabei erfolgt die Umsetzung zum gewünschten 5-Chlorimidazol-4-carbaldehyd beispielsweise mit Phosphoroxychlorid in Gegenwart von N,N-Dimethylformamid.

Zweckmässig liegt das Molverhältnis der Reaktanden, gegebenenfalls 2-substituiertes 5-Chlorimidazol zu Phosphoroxychlorid zu N,N-Dimethylformamid, im Bereich zwischen 1:1:1 und 1:5:5, bevorzugt bei ungefähr 1:3:3.

Die Umsetzungstemperatur liegt zweckmässig zwischen 50° und 130°C.

Gegebenenfalls kann in Gegenwart eines zusätzlichen inerten Lösungsmittels ,beim Eintopfverfahren vorteilhaft im Lösungsmittel der ersten Stufe, gearbeitet werden.

Die Isolierung des resultierenden gegebenenfalls 2-substituierten 5-Chlorimidazol-4-carbaldehyds aus dem Reaktionsgemisch erfolgt auf fachmännische Weise vorteilhaft durch dessen Extraktion mit einem geeigneten Lösungsmittel.

### Beispiele:

### Herstellung von 2-n-Butyl-3,5-dihydroimidazol-4-on

Zu einer Lösung von 10,1 g (0,25 mol) Natriumhydroxid in Methanol bei 0°C wurden 31,71 g (0,25 mol) Glycinmethylesterhydrochlorid zugegeben. Nach 15 Minuten wurden zur weissen Suspension 126,5 g einer 22,8%igen Lösung von Pentanimidsäuremethylester in Chlorbenzol während 5 Minuten zugetropft. Die hellgelbe Suspension wurde 4 Std. bei Raumtemperatur gerührt und mit Chlorbenzol (100 ml) verdünnt. Das Methanol wurde bei einer Temperatur von 26°C und einem Druck von 30 - 50 mbar abdestilliert,und die orange Suspension wurde mit Methylenchlorid (100 ml) verdünnt und anschliessend filtriert. Nach Entfernung des Lösungsmittels vom Filtrat wurden 34,08 g (97%) der Titelverbindung (Gehalt >95% nach GC und ¹H-NMR) erhalten.

### Herstellung von 2-n-Butyl-5-chlor-1H-imidazol

2-n-Butyl-3,5-dihydroimidazol-4-on (14,02 g, 0,1 mol) wurde portionenweise während 15 Minuten zu POCl₃ (50 ml) bei 95°C gegeben. Die Lösung wurde 2 Std. bei 100°C erhitzt, gekühlt und auf 400 g Eis gegossen. Das Gemisch wurde mit 255 ml 30%iger Natronlauge auf pH 7 gestellt und dreimal mit je 500 ml Essigester extrahiert. Die vereinigten organischen Phasen wurden mit MgSO₄ getrocknet, filtriert und am Rotavapor eingeengt. Nach Reinigung des Rückstands mittels Säulenchromatographie wurde die Titelverbindung (5,52 g, 34,7%) in hoher Reinheit (>98% nach GC und ¹H-NMR) erhalten.
- Smp. 85° - 87°C:
- ¹H-NMR (CDCl₃) δ: 0,91 (3H, t, J = 7,5 Hz),
1,36 (2H, sextett, J = 7,5 Hz),
1,68 (2H, q, J = 7,5 Hz),
2,70 (2H, t, J = 7,5 Hz),
6,83 (1H, s),
10,65 (1H, br s).

### Herstellung von 2-n-Butyl-5-chlorimidazol-4-carbaldehyd aus 2-n-Butyl-5-chlor-1H-imidazol

Zu einer auf 95°C erwärmten Lösung von 2-n-Butyl-5-chlor-1H-imidazol (1,60 g, 10 mmol) in POCl₃ (3,07g, 20 mmol) und Chlorbenzol (20 ml) wurde N,N-Dimethylformamid (1,46g, 20 mmol) zugetropft. Das Gemisch wurde während 3,5 Std. bei 98°C gerührt. Danach wurden weitere Portionen von POCl₃ (1,53 g, 10 mmol) und von N,N-Dimethylformamid (0,73 g, 10 mmol) zugetropft. Nach weiteren 2,5 Std. bei 98°C wurde das Gemisch gekühlt und auf Eis (40 g) gegossen. Nach 15 Minuten wurde das Gemisch mit 11 ml 30%iger Natronlauge auf pH 7 gestellt und dreimal mit je 100 ml Essigester extrahiert. Die vereinigten organischen Phasen wurden mit MgSO₄ getrocknet, filtriert und eingeengt. Die Titelverbindung wurde in einer Ausbeute von 1,3 g (70%) erhalten.

## Patentansprüche

1. Verfahren zur Herstellung von gegebenenfalls 2-substituierten 5-Chlorimidazolen der allgemeinen Formel 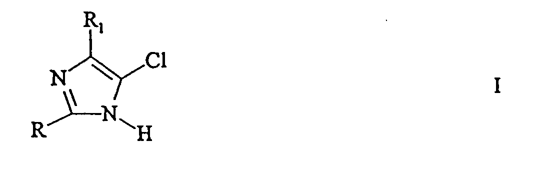 worin R Wasserstoff, eine geradkettige oder verzweigte C₁-C₆-Alkylgruppe, eine geradkettige oder verzweigte C₂-C₆-Alkenylgruppe, eine Cyclopropyl-, Cyclobutyl-, Cyclopentyl- oder Cyclohexylgruppe, oder eine gegebenenfalls mit Halogenatomen, Alkyl-, Nitro- und/oder Aminogruppen substituierte Benzylgruppe oder Phenylgruppe bedeutet und R₁ Wasserstoff, eine geradkettige oder verzweigte C₁-C₆-Alkylgruppe, eine Cyclopropyl-, Cyclobutyl-, Cyclopentyl- oder Cyclohexylgruppe, oder eine gegebenenfalls mit Halogenatomen, Alkyl-, Nitro- und/oder Aminogruppen substituierte Benzylgruppe oder Phenylgruppe bedeutet, dadurch gekennzeichnet, daß in einer ersten Stufe ein Glycinesterhydrohalogenid der allgemeinen Formel 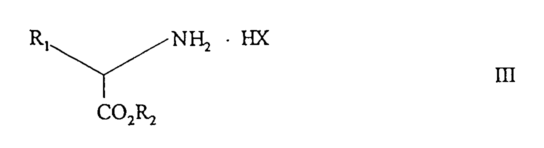 worin R₁ die oben genannte Bedeutung hat, R₂ eine Alkylgruppe bedeutet und X für ein Halogenatom steht, mit einem Imidsäureester der allgemeinen Formel 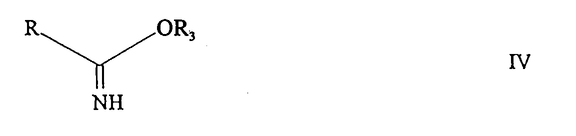 worin R die oben genannte Bedeutung hat und R₃ eine Alkylgruppe bedeutet, in Gegenwart einer Base zum entsprechenden 3,5-Dihydroimidazol-4-on der allgemeinen Formel 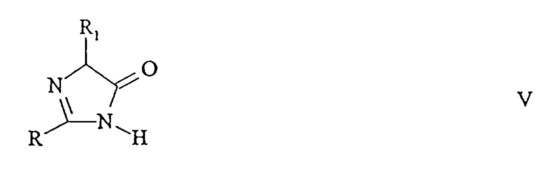 worin R und R₁ die oben genannte Bedeutung haben, umgesetzt wird und in einer zweiten Stufe zum Endprodukt chloriert wird.

2. Verfahren nach Anspruch 1, dadurch gekennzeichnet, daß als Base in der ersten Stufe ein Alkalihydroxid oder ein Alkalialkoholat verwendet wird.

3. Verfahren nach Anspruch 1 oder 2, dadurch gekennzeichnet, daß die Umsetzung in der ersten Stufe in einem pH-Bereich zwischen 7 und 12 durchgeführt wird.

4. Verfahren nach einem der Ansprüche 1 bis 3, dadurch gekennzeichnet, daß die Reaktionsteilnehmer der ersten Stufe, nämlich Glycinesterhydrohalogenid, Imidsäureester und Base, im stöchiometrischen Molverhältnis 1:1:1 umgesetzt werden.

5. Verfahren nach einem der Ansprüche 1 bis 4, dadurch gekennzeichnet, daß die Umsetzungstemperatur in der ersten Stufe zwischen -20°C und 50°C liegt.

6. Verfahren nach einem der Ansprüche 1 bis 5, dadurch gekennzeichnet, daß die Chlorierung in der zweiten Stufe mit Thionylchlorid oder Phosphoroxychlorid durchgeführt wird.

7. Verfahren nach Anspruch 6, dadurch gekennzeichnet, daß die Chlorierung mit Phosphoroxychlorid bei einer Umsetzungstemperatur zwischen 20°C und 110°C durchgeführt wird.

8. 2-Substituiertes 5-Chlorimidazol der allgemeinen Formel 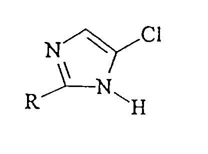 worin R n-Butyl, 2-Butenyl oder 3-Butenyl bedeutet.

## Claims

1. A process for the preparation of optionally 2-substituted 5-chlorimidazoles of the general formula 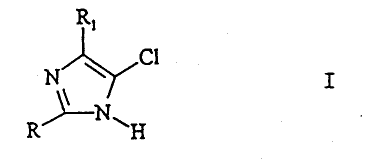 wherein R denotes hydrogen, a straight or branched C₁-C₆ alkyl group, a straight or branched C₂-C₆ alkenyl group, a cyclopropyl-, cyclobutyl-, cyclopentyl- or cyclohexyl group or a benzyl or phenyl group optionally substituted with halogen atoms, alkyl, nitro and/or amino groups and R₁ denotes hydrogen, a straight or branched C₁-C₆ alkyl group, a cyclopropyl-, cyclobutyl-, cyclopentyl- or cyclohexyl group or a benzyl group or phenyl group optionally substituted with halogen atoms, alkyl, nitro and/or amino groups, characterised in that in a first step a glycine ester hydrohalide of the general formula 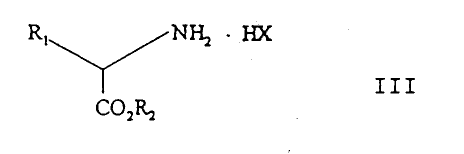 wherein R₁ has the meaning defined above, R₂ denotes an alkyl group and X stands for a for a halogen atom, is reacted in a first step with an imido-acid ester of the general formula 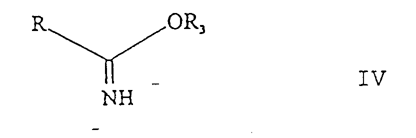 wherein R has the above-mentioned meaning and R₃ denotes an alkyl group in the presence of a base to give the corresponding optionally substituted 3,5-dihydroimidazol-4-one of the general formula 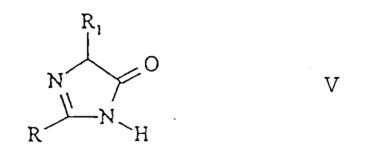 wherein R and R₁ have the said meanings and is chlorinated in a second step to give the end product.

2. A process according to claim 1, characterised in that an alkali hydroxide or an alkali alkoxide is used as base in the first step.

3. A process according to claim 1 or 2, characterised in that in the first step the reaction is carried out in a pH range between 7 and 12.

4. A process according to claims 1 to 3, characterised in that the reaction components of the first step, that is the glycine ester hydrohalide, the imidocarboxylic ester and the base are reacted in a stoichiometric ratio of 1:1:1.

5. A process according to claims 1 to 4, characterised in that in the first step the reaction temperature lies between -20°C and 50°C.

6. A process according to claims 1 to 5, characterised in that in the second step the chlorination is carried out with thionyl chloride or phosphorus(V) oxychloride.

7. A process according to claim 6, characterised in that the chlorination is carried out with phosphorus(V) oxychloride at a temperature between 20°C and 110°C.

8. A 2-substituted 5-chlorimidazole of the general formula 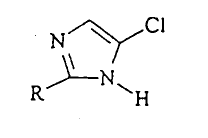 in which R is n-butyl, 2-butenyl or 3-butenyl.

## Revendications

1. Procédé pour la préparation de 5-chloroimidazoles éventuellement substitués sur la position 2, de la formule générale 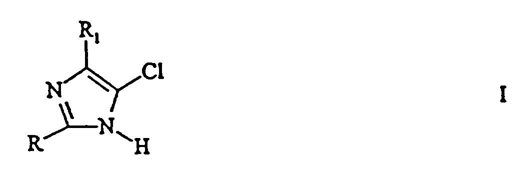 dans laquelle R signifie hydrogène, un groupe alkyle linéaire ou ramifié C₁-C₆, un groupe alcényle linéaire ou ramifié C₂-C₆, un groupe cyclopropyle, cyclobutyle, cyclopentyle ou cyclohexyle, ou un groupe phényle ou un groupe benzyle éventuellement substitué par des atomes halogène, des groupes alkyle, nitro et/ou amino et R₁ signifie hydrogène, un groupe alkyle linéaire ou ramifié C₁-C₆, un groupe cyclopropyle, cyclobutyle, cyclopentyle ou cyclohexyle, ou un groupe phényle ou benzyle éventuellement substitué par des atomes halogène, des groupes alkyle, nitro et/ou amino, caractérisé en ce que dans une première étape, on met en réaction un hydrohalogénure d'ester de glycine de la formule générale 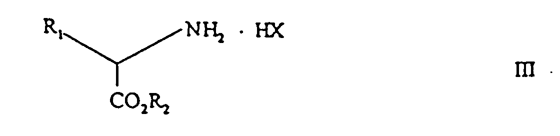 dans laquelle R₁ a la signification sus-mentionnée, R₂ signifie un groupe alkyle et X un atome halogène, avec un ester de l'acide imidique de la formule générale 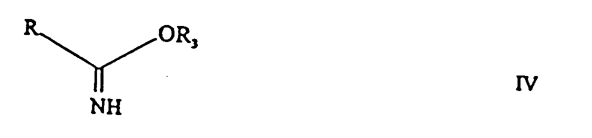 dans laquelle R a la signification sus-mentionnée et R₃ signifie un groupe alkyle, en présence d'une base pour être transformé en la 3,5-dihydro-imidazol-4-one correspondante de la formule générale 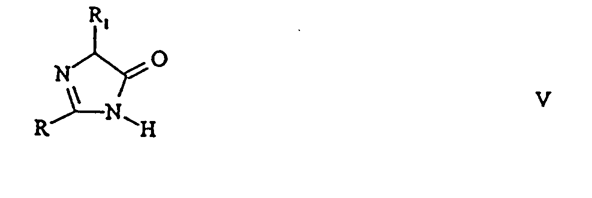 dans laquelle R et R₁ ont la signification sus-mentionnée et en ce que dans une deuxième étape, on procède à la chloration pour donner le produit final.

2. Procédé selon la revendication 1, caractérisé en ce qu'en tant que base, on utilise dans la première étape un hydroxyde alcalin ou un alcoolat alcalin.

3. Procédé selon la revendication 1 ou 2, caractérisé en ce que dans la première étape, la réaction est conduite dans une plage de pH entre 7 et 12.

4. Procédé selon l'une des revendications 1 à 3, caractérisé en ce que les participants à la réaction de la première étape, en l'occurrence l'hydrohalogénure d'ester de glycine, l'ester de l'acide imidique et la base, sont convertis dans un rapport molaire stoechiométrique 1:1:1.

5. Procédé selon l'une des revendications 1 à 4, caractérisé en ce que la température de réaction dans la première étape se situe entre -20 et 50°C.

6. Procédé selon l'une des revendications 1 à 5, caractérisé en ce que la chloration dans la deuxième étape s'effectue avec du chlorure de thionyle ou de l'oxychlorure de phosphore.

7. Procédé selon la revendication 6, caractérisé en ce que la chloration avec l'oxychlorure de phosphore est conduite à une température de réaction entre 20 et 110°C.

8. 5-chloro-imidazole substitué en position 2 de la formule générale 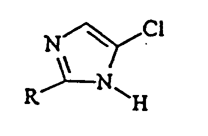 dans laquelle R signifie n-butyle, 2-butényle ou 3-butényle.
